Europäisches Patentamt

European Patent Office

Office européen des brevets

(11) Publication number: **0 506 159 A1**

# EUROPEAN PATENT APPLICATION

(21) Application number: **92200589.7**

(22) Date of filing: **02.03.92**

(51) Int. Cl.5: **C12P 7/62**, B01D 61/36,
C07C 69/24, C07C 67/03

(30) Priority: **19.03.91 EP 91302341**

(43) Date of publication of application:
**30.09.92 Bulletin 92/40**

(84) Designated Contracting States:
**AT BE CH DE DK ES FR GB GR IT LI NL PT SE**

(71) Applicant: **UNICHEMA CHEMIE BV**
**Buurtje 1**
**NL-2802 BE Gouda(NL)**

(72) Inventor: **Kemp, Roger Alan**
**18 Kinglass Road**
**Spital Nr. Bebington, Wirral L63 9AJ(GB)**
Inventor: **Macrae, Alasdair Robin**
**Ivy Cottage**
**Newton Blossom Ville, Bedford MK43**
**8AN(GB)**

(74) Representative: **Hartong, Richard Leroy et al**
**Unilever N.V. Patent Division P.O. Box 137**
**NL-3130 AC Vlaardingen(NL)**

(54) **Esterification process.**

(57) A process is described for the esterification of a
carboxylic acid, such as myristic acid, with an al-
cohol, such as isopropyl alcohol, wherein the acid
and alcohol are contacted with an esterification en-
zyme, such as a lipase, and the water formed in the
reaction is removed by pervaporation.

This invention relates to a process for the esterification of an acid with an alcohol.

It is known in the art to carry out esterification reactions in the presence of an esterification catalyst. The catalyst is usually an acidic catalyst such as s sulphonic acid, a base such as an alkali hydroxide or an alkali alcoholate, a metal oxide or a metal alkylate. Such processes are disclosed inter alia in DE-A-2503195 (Henkel) and EP-A-334154 (Henkel). Such esterification reactions take place at a high temperature, such as at 120-145°C or higher. While a high temperature may be advantageous in the removal of the water formed in the esterification reaction, it is expensive particularly in terms of energy consumption.

Esterification reactions have been proposed in our co-pending European patent applications 89202659.2 and EP-A-424,130 filed 20 October 1989 and 18 October 1990 in which an enzyme catalyst, such as lipase, is used. Enzymatic esterification takes place at a lower temperature and therefore with a lower energy input, but there remains the need to remove the water formed in the reaction from the product.

Distillation under reduced pressure has been used to remove water from the reaction mixtures, but the requirement to supply heat to the reaction mixture for distillation can cause enzyme catalyst inactivation. Some carboxylic acid and alcohol reactants are immiscible at the temperatures used for enzyme catalysed esterification and in these cases, to achieve an acceptable reaction rate, addition of an inert volatile solvent in which the reactants have mutual solubility is required. Removal of water by distillation from reaction mixtures containing volatile solvents is difficult because of co-distillation of the solvent with the water.

We have now surprisingly discovered that the technique of pervaporation can be used to remove the water formed in enzymatic esterification without the need for high temperatures.

Thus, according to the invention there is provided a process for the esterification of a carboxylic acid with an alcohol wherein a mixture of the acid and the alcohol is contacted with an esterification enzyme and the water formed in the reaction is removed by pervaporation. Optionally, an inert solvent may be added to the mixture of the acid and the alcohol.

Pervaporation is a known technique. In European Patent EP-A-210,055 (BP Chemicals Limited) there is described a catalytic esterification process for producing liquid esters, which is carried out in a reactor provided with a pervaporation membrane. Thereby, continual removal of reaction products (such as water) is possible. In this process, an acidic catalyst is used and the esterification is carried out at a temperature of 20-120°C.

The pervaporation membrane used in this process has to not only maintain its stability at the working temperature but also has to have adequate resistance to the acidic environment of the esterification reaction.

In European Patent Application EP-A-372,683 (Texaco Development Corp.) it has been proposed to dehydrate organic oxygenates, such as esters (like propylene glycol monostearate, ethyl acetate) by pervaporation, using polyvinyl alcohol cross-linked with aliphatic polyaldehyde as the separating layer. Finally, in United States Patent Specification US-A-2,956,070 (The American Oil Comp.) a catalytic esterification process has been described, in which the reaction is performed in a reactor provided with a pervaporation membrane. Cation exchange material in sheet or granular form or mild acid catalysts are used, and the temperature is fairly high (100°-200°C), whereas at 150°C a pressure of 5.1 atm over-pressure is used. In essence, the disclosure of US-A-2,956,070 is the same as of EP-A-210,055, although in the former document higher temperatures and pressures are used.

We have now discovered that pervaporation can also be used to remove the water formed in enzyme esterification reactions and, since no acid catalyst need be present and since the esterification reaction temperature may be low, the limitations placed upon the nature of the pervaporation membrane are reduced.

The esterification reaction is preferably carried out at from 50° to 70°C such as about 60°C. The carboxylic acid and the alcohol (and, if required, the inert solvent) are preferably mixed before contacting the enzyme. While a stoichiometic mixture may be used, it is of advantage if the more volatile reactant be present in some excess. The molar ratio of the more volatile to the less volatile reactant maybe from 1:1 to 4.5:1, such as from 1.5:1 to 4:1. Thus, when isopropyl alcohol is to be esterified with myristic acid we have found a ratio of about 3.5:1 to be suitable.

The mixture is preferably heated to the desired esterification temperature before being contacted with the enzyme.

The carboxylic acid is preferably a monocarboxylic acid. It may contain 2 to 24, but preferably from 6 to 20 carbon atoms in the molecule. The carboxylic acid may contain one or more double bonds in the molecule and may be branched or straight chain. Suitable carboxylic acids include caprylic acid, capric acid, lauric acid, myristic acid, palmitic acid, stearic acid, iso-stearic acid, oleic acid, linoleic acid etc. The process is especially suitable for carboxylic acids derived from natural sources, such as vegetable and animal fats.

The alcohol is preferably a monoalcohol, although polyols may also be used. The alcohol may contain 1 to 24 carbon atoms in the molecule, especially suitable being the more volatile $C_1$-$C_5$ alcohols or the less volatile $C_{12}$-$C_{24}$ alcohols. The carbon chain in the alcohol may be saturated or unsaturated, straight or branched chain. Preferably in the alcohol is a primary alcohol. Suitable alcohols include ethanol, propanol, isopropanol, butanols, pentanols, ethylene glycol, lauryl alcohol, myristyl alcohol, cetyl alcohol, tetramethylene glycol, pentamethylene glycol, glycerol, sorbitol, glucose and other mono- and disaccharides and their derivatives. The process is especially suitable for alcohols derived from natural sources, such as from vegetable oils and animal fats.

The choice of the optionally used inert solvent depends on the properties of the enzyme and reactants used, the solvent being selected to ensure that the reactants have mutual solubility and enzyme catalyst activity is not impaired. Suitable inert solvents which may be used for the enzymatic esterification include: alkanes such as petroleum ether, hexane, and iso-/octane; aromatic hydrocarbons, such as toluene and benzene; halogenated hydrocarbons, such as chloroform, carbon tetrachloride and trichloroethane; ethers, such as diethyl ester, di-/isopropyl ether and dibutyl ether; ketones, such as methylethylketone, methylpropylketone and diethylketone; tertiary alcohols, such as t-butanol and t-amylalcohol; nitromethane; tributyl phosphate, or mixtures thereof. The esterification enzyme preferably comprises a lipase, and may indeed consist only of lipase. The lipase can be obtained by culturing a suitable micro-organism and subsequently isolating the enzyme. Suitable micro-organisms for this purpose belong to the genuses Micor, Aspergillus, Rhizopus, Pseudomonas, Candida, Humicola, Thermomyces and Pencillium.

The enzyme may be supported on a carrier material selected from hydrophobic materials and ion-exchange resins. Particulate carrier materials are preferred, especially those having a particle size from 100 to 2000 $\mu$m. The carrier material may also be in the form of a membrane. The carrier material is preferably porous, with an average pore diameter greater than 50 nanometers. Suitable supported enzymes are described in EP-A-322,213 (Unilever), US 4 798 793 and US 4 818 695 (Novo).

The pervaporation takes place across a pervaporation membrane. This membrane may be of any of the commercially available varieties, provided that they have adequate resistance to the esterification reaction mixture. The pervaporation membrane is typically a multilayer arrangement - an active outer layer often a modified PVA membrane, a porous backing layer and an outer layer than confers support to the other two layers. Alternatively, the active layer can be attached to the inside surfaces of a ceramic microfiltration module. Suitable membranes include those manufactured by GFT Ingenieur Büro für Industrie Anlagen Planung, which e.g. may consist of a polyvinyl alcohol active layer, cast onto a layer of polyacrylonitrile, which in turn is cast onto a support layer of a non-woven polyester (like Terylene; Trade Mark), Nafion (Trade Mark) membranes (which may be perfluorosulphonic acid cation exchange polymer in the form of supported or non-supported film) and those manufactured by Kalsep Ltd, Langley, Berkshire, UK.

In order to achieve optimum results from the process according to the invention, the pressure conditions are important. Where a pervaporation temperature of 50-80°C is used, the low pressure side of the membrane may suitably be at a pressure of 10-300 mbar, such as about 20 mbar.

A suitable pervaporation device is in the form of a stack of flat plates with inlet and outlet ports. These plates have a sealing gasket around the perimeter which allows compression and hence sealing against the membrane. The membrane is suspended across the void formed by two opposing recesses in the plates. Permeate passes through the membrane into the void on the far side, i.e. the low pressure side, at which point it vaporises as a result of the temperature/vacuum and then passes out of the outlet port for subsequent condensation. The retentate, which is the partially reacted ester and, optionally, an inert solvent, does not enter the membrane and passes out of the apparatus via another opposing outlet port.

A suitable apparatus of this description is the PV separator, from GFT (Gesellschaft für Trenntechnik mbH) Neunkirchen-Heinitz, Germany.

Alternatively, the pervaporation device can be in the form of a circular tube bundle with the membrane disposed on the inside of the tube wall.

A suitable apparatus for carrying out the process of the invention comprises an esterification reactor, means for feeding the carboxylic acid and the alcohol and, if required, an inert solvent to the esterification reactor, an esterification enzyme supported within the reactor for contact with the mixture of the carboxylic acid and the alcohol fed thereto, a pervaporation device connected to the esterification reactor to receive the esterification reaction product mixture therefrom and comprising a pervaporation membrane and means for removing water formed in the esterification process from the pervaporation device.

It will be beneficial to include a heating device, such as a heat exchanger, between the reactor and the pervaporation device to raise the temperature of the esterification mixture from the esterification temperature to the pervaporation temperature.

In a modification of this arrangement, the product leaving the pervaporation device may be passed to one or more further esterification reactors and/or recycled, to increase the overall yield.

The invention will now be illustrated by the following non-limiting eamples.

EXAMPLE I

Isopropyl alcohol which had been passed through an ion-exchange bed to eliminate any trace levels of metal ions, was mixed with myristic acid at a molar ratio of 3.5:1 and heated to 65°C. The mixture was passed to an esterification reactor containing a fixed catalyst bed. The catalyst was Lipase B. In this reactor, conversion of 70 to 80% occured and the product contained about 3.2% water. The product was heated to 80°C and passed to a pervaporation device containing a GFT membrane. The device was operated at 20 mbar vacuum. The water level in the product fell to about 0.4%. The product was then cooled to 65°C and passed to second and third esterification reactors, of similar construction to the first. Some recycling at this point appeared to be advantageous. At this point 98.5% conversion was achieved and the product contained to 1.5% water. The product was again heated to 80°C and passed to a second pervaporation device, similar in construction to the first, where the water level in the product fell to about 0.2%. The product was then cooled to 65°C and passed to a final esterification reactor where a conversion of 99.8% or more was achieved. Finally, the product was bleached using fixed bed carbon and deodorised with nitrogen.

By this process much greater reaction rates are achieved than with a conventional acid catalyst. This results in lower plant residence time, higher yields and lower capital and running costs.

EXAMPLE II

An isopropyl myristate reaction mixture was prepared at 60°C by the passage of isopropanol/myristic acid mixture at mole ratio 3.5:1 over a fixed bed of lipase B present on a support medium. The resulting product contained 70% ester and 2.5% water. This reaction mixture was then pervaporated at temperatures of 90°C and 80°C on a GFT acid resisting membrane. This membrane produced flux rates of 5 $kg/m^2/hr$ and 3.85 $kg/m^2/hr$ respectively, and the subsequent water content in the reaction mixture was 0.2%. This dehydrated reaction mixture was then further reacted over a lipase fixed bed to obtain a conversion of 97.5%. This conversion percentage can of course be increased by repeating the esterification/pervaporation treatment.

EXAMPLE III

A reaction mixture of isopropyl myristate as obtained by a process as described in the first step of Example II containing 65% ester, 3% water, 8% fatty acid and 24% residual isopropanol was pervaporated on a standard GFT membrane. This produced a reaction mixture, when pervaporated at 80°C, with a water content of 0.15%, and a flux rate of 464 $kg/m^2/hr$ was obtained. This reaction mixture was subsequently re-esterified over a lipase B bed to an ester content of 99.04%. No apparent damage to the standard GFT pervaporation membrane was reported.

**Claims**

1. A process for the esterification of a carboxylic acid with an alcohol wherein a mixture of the acid and the alcohol are contacted with an esterification enzyme and the water formed in the reaction is removed by pervaporation.

2. A process according to claim 1, wherein the mixture of the carboxylic acid and the alcohol is contacted with the esterification enzyme at a temperature below 80°C, preferably at a temperature of from 60 to 70°C.

3. A process according to claim 1, wherein the esterification enzyme comprises a lipase.

4. A process according to claim 1, wherein an inert solvent is added to the mixture of the acid and the alcohol before esterification.

5. A process according to claim 1, wherein the water formed in the reaction is removed by pervaporation at a temperature of less than 98°C, preferably at a temperature of from 70 to 90°C.

6. An apparatus for the esterification of a carboxylic acid with an alcohol comprising an esterification reactor, means for feeding the carboxylic acid and the alcohol and, optionally, an inert solvent to the esterification reactor, an esterification enzyme supported within the reactor for contact with the mixture of the carboxylic acid and the alcohol fed thereto, a pervaporation device connected to the esterification reactor to receive the esterification reaction product mixture therefrom and comprising a pervaporation membrane and means for removing water formed in the esterification process from the pervaporation device.

7. An apparatus according to claim 5, wherein the pervaporation membrane is selected from PVA pervaporation membranes and an acid-resisting pervaporation membranes.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,Y | US-A-2 956 070 (J.F. JENNINGS ET AL.) <br> * column 3, line 66; claims 1,2 * | 1-3,5,6 | C12P7/62 <br> B01D61/36 <br> C07C69/24 |
| Y | EP-A-0 383 405 (UNICHEMA CHEMIE) <br> * page 2, line 44 - page 3, line 39 * | 1-3,5,6 | C07C67/03 |
| D,A | EP-A-0 372 683 (TEXACO DEVELOPMENT) <br> * claims 1,10 * | 1,7 | |
| A | PATENT ABSTRACTS OF JAPAN <br> vol. 11, no. 115 (C-415)(2562) 10 April 1987 <br> & JP-A-61 257 191 ( MEITO SANGYO ) 14 November 1986 <br> * abstract * | 1-3 | |
| A | FR-A-2 646 101 (INSTITUT FRANCAIS OU PETROLE ET CARGOCAIRE FRANCE) <br> * page 10, line 4 - page 10, line 13 * | 1,6 | |
| D,A | US-A-4 798 793 (P. EIGTVED) <br> * column 5, line 5-6 * | 1,4 | TECHNICAL FIELDS SEARCHED (Int. Cl.5) |
| A | EP-A-0 190 651 (HENKEL) <br> * page 2, line 7 - page 2, line 14 * | 1,4 | C12P <br> B01D |
| A | AGRICULTURAL AND BIOLOGICAL CHEMISTRY. <br> vol. 46, no. 2, February 1982, TOKYO JP <br> pages 405 - 409; <br> C.W. SEO ET AL.: 'Synthesis of fatty acid esters by Corneybacterium sp. S-401' <br> * page 405, column 1, paragraph 2 * | 1,4 | C07C |

The present search report has been drawn up for all claims

| Place of search | Date of completion of the search | Examiner |
|---|---|---|
| BERLIN | 02 JULY 1992 | PROBERT C. |

EPO FORM 1503 03.82 (P0401)